# EUROPÄISCHE PATENTSCHRIFT

(11) **EP 2 529 762 B1**
(45) Veröffentlichungstag und Bekanntmachung des Hinweises auf die Patenterteilung: **16.10.2019**
(21) Anmeldenummer: 12004018.3
(22) Anmeldetag: 23.05.2012
(51) Int. Cl.: A61L 27/16, A61K 6/083

(54) **NACH HÄRTUNG BRUCHRESISTENTES PROTHESENBASISMATERIAL, ERHALTEN AUS AUTOPOLYMERISIERENDEN ODER KALTPOLYMERISIERENDEN ZUSAMMENSETZUNGEN**
PROSTHETIC BASE MATERIAL THAT IS BREAK-RESISTANT AFTER HARDENING, DERIVED FROM AUTOPOLYMERISING OR COLD POLYMERISING COMPOUNDS
MATIÈRE DE BASE DE PROTHÈSE RÉSISTANT À LA RUPTURE APRÈS DURCISSEMENT, OBTENUE À PARTIR DE COMPOSITIONS À AUTOPOLYMÉRISATION OU À POLYMÉRISATION À FROID

(30) Priorität: 30.05.2011 DE 102011106816
(43) Veröffentlichungstag der Anmeldung: 05.12.2012
(73) Patentinhaber: Kulzer GmbH, 63450 Hanau (DE)
(72) Erfinder: RUPPERT, Klaus, 63477 Maintal (DE); KERSCHER, Kevin, 61297 Neu-Anspach (DE)
(74) Vertreter: Bendele, Tanja

(56) Entgegenhaltungen:
- EP-A2- 0 995 421
- EP-A2- 1 702 633
- EP-A2- 1 923 037
- WO-A2-2007/007065

## Beschreibung

Die Erfindung betrifft nach Härtung bruchresistentes Prothesenbasismaterial, erhalten aus autopolymerisierenden oder kaltpolymerisierenden Zusammensetzungen.

### Hintergrund

Gehärtete Prothesenwerkstoffe neigen bei der Ausbettung aus dem Einbettmaterial zum Abplatzen von dünn auslaufenden Bereichen. Ferner fallen Prothesen aufgrund der eingeschränkten Motorik der in der Regel älteren Prothesenträger manchmal auf einen harten Untergrund (Fliesen, Waschbecken), so dass es hier ebenfalls zu Abplatzem kommen kann. Diese Abplatzer bedeuten Zusatzarbeit und -kosten für das Dentallabor und sind deshalb unerwünscht.

Aus diesen Gründen besteht vom Markt her die Anforderung nach einem Prothesenwerkstoff, welcher diesen kurzzeitigen Energieeintrag ohne Materialschädigung toleriert, sogenannte High Impact-Werkstoffe.

Produkte, welche diese Anforderungen erfüllen, sind schon seit einiger Zeit auf dem Markt, sie sind allerdings alle der Gruppe der heißhärtenden Prothesenwerkstoffe zuzuordnen.

Zum Erzielen einer hohen Bruchresistenz kommen derzeit verschiedene Technologien zur Anwendung, beispielsweise die in EP1923037A2 beschriebenen, nämlich
i. Kern-Schale Partikel und
ii. spezielle elastifizierende Moleküle wie z.B. Butadien-Copolymere oder Butadien-Acrylnitril-Copolymere.

Zu i): Aufgrund der für Auto- oder Kaltpolymerisate geforderten kurzen Anquellzeit der Perlen sind Kern-Schale-Partikel in der Regel ungeeignet zur Erzielung einer hohen Bruchresistenz. Die Zeit reicht nicht aus, um die Perlen genügend anquellen zu lassen.

Zu ii): Ein Problem besteht darin, dass in vielen elastifizierenden synthetischen Kautschuk-Molekülen Nitril-Gruppen enthalten sind, die zum Vergilben neigen, typischerweise in Gegenwart von oxidierenden Substanzen wie z.B. Peroxiden oder Luftsauerstoff.

EP1923037 A offenbart ein bruchresistentes Prothesenmaterial auf der Basis von Methylmethacrylat, dem flüssige Butadien-(meth)acryl- oder -(meth)acrylnitril-Oligomere als Modifikatoren zugesetzt werden. Das Material wird durch Auto- oder Kaltpolymerisation und ggf. im Gemisch mit einem Polymerpulver oder Perlpolymerisat erhalten.

### Aufgabe

Es ist ein kaltpolymerisierendes, nach Härtung bruchresistentes Prothesenbasismaterial anzugeben. Der ausgehärtete Werkstoff sollte eine gegenüber einem Referenzwerkstoff ohne Additiv erhöhte Bruchzähigkeit von > 1,8 MPa*m^{1/2} und erhöhte Brucharbeit von > 400 J/m² aufweisen. Außerdem sollte er möglichst auch insofern farbstabll seln, als er nicht bzw. kaum vergilbt. Anders ausgedrückt soll er nahezu farblos bleiben und folglich farbstabil sein, wobei das Ausmaß der Farbstabilität nur eine Veränderung des Gelbwerts um eine Einheit sein soll, wenn der Werkstoff in vollentsalztem Wasser 8 Wochen bei 50°C gelagert wird. Es ist weiterhin anzustreben, dass die Resultate des sogenannten Suntests nach ISO 20795 nicht durch neuartige Zusätze negativ beeinfluSSt werden.

### Die Erfindung im Einzelnen

Erfindungsgemäß wird die Aufgabe durch bruchresistente Prothesenwerkstoffe bzw. nach Härtung bruchresistentes Prothesenbasismaterial gelöst, die aus autopolymerisierenden oder kaltpolymerisierenden Zusammensetzungen erhalten werden, die enthalten:
(A) eine flüssige Monomerkomponente,
(B) eine pulverförmige Komponente, die vorzugsweise ein oder mehrere Perlpolymerisat(e),
(C) mindestens einen Initiator für die Auto- oder Kaltpolymerisation,
wobei in (A) oder/und (B) erfindungsgemäß mindestens ein Mitglied der Gruppen der aliphatischen Urethanacrylate und der aliphatischen Urethanmethacrylate vorhanden ist/sind, insbesondere in Komponente (A) flüssiges aliphatisches Urethandiacrylat-Harz, das sich aus aliphatischen Urethan- und Acrylsegmenten zusammensetzt. Bevorzugt sind die Zusammensetzungen frei von elastifizierendem synthetischen Kautschuk.

Dabei ergeben sich nach dem Aushärten vorzugsweise die Materialwerte Bruchzähigkeit von > 1,8 MPa*m^{1/2} und Brucharbeit von > 400 J/m². Besonders bevorzugt weist das ausgehärtete Material nach 8 Wochen Lagern in vollentsalztem Wasser (VE-Wasser) bei 50°C eine Erhöhung im Gelbwert (b-Wert) von maximal einer Einheit auf.

Die Erfindung betrifft somit Prothesenwerkstoffe gemäß Anspruch 1. Vorteilhafte Ausgestaltungen und Verwendungen sind den weiteren Ansprüchen zu entnehmen.

Es versteht sich, dass In dieser Beschreibung unter Prothesenwerkstoff, Prothesenmaterial und Prothesenbasismaterial von Fall zu Fall die Ausgangszusammensetzung vor der Härtung oder das gehärtete Material beziehungsweise der gehärtete Werkstoff mit seinen Eigenschaften gemeint ist.

Unter aliphatischen Urethan(meth)acrylaten sind im Sinne der Erfindung bevorzugt Polymere mit aliphatischen Urethansegmenten und (Meth)Acrylatsegmenten in der Polymerstruktur zu verstehen. Dass diese die Eigenschaften der Prothesenwerkstoffe derart positiv beeinflussen, war in diesem Ausmaß nicht zu erwarten. Insbesondere kann ganz auf synthetische Kautschuke verzichtet werden.

Urethanacrylate und Urethanmethacrylate sind in der Regel farblos, farbstabil, transparent, ohne Eigengeruch und beeinträchtigen die Eigenschaften der Grundrezeptur nicht. Die Erfindung betrifft somit auch die Verwendung eines aliphatischen Urethanacrylates oder Urethanmethacrylates, insbesondere z.B. Albiflex® XP 6/1166 der Fa. Nano Resins / Geesthacht, Deutschland, als Modifikator in auto- oder kaltpolymerisierenden Prothesenwerkstoffen.

Bei Albiflex® XP 6/1166 handelt es sich gemäß technischem Datenblatt der obengenannten Firma von Januar 2010 um das folgende Versuchsprodukt

"Albiflex XP 6/1166 ist ein neuartiges aliphatisches Urethandiacrylat-Harz, das zur Formulierung von Hochleistungselastomeren vorgesehen ist. Albiflex XP 6/1166 ist bei Umgebungstemperatur flüssig und härtet mit allen radikalbildenden Photoinitiatoren ebenso wie mit Peroxiden. Es kann mit allen gebräuchlichen Gieß- oder Beschichtungstechniken verarbeitet werden. Die besonders eingestellte Struktur von Albiflex XP 6/1166, das sich aus aliphatischen Urethan- und Acrylsegmenten zusammensetzt, sorgt für eine hervorragende Kombination von Eigenschaften:
- viel größere Festigkeit als herkömmliche Diacrylate;
- hervorragende Haftung auf den meisten Kunststoffsubstraten;
- bessere Wetterfestigkeit als Polyesteracrylate;
- hervorragende Kompatibilität mit Nanocryl®-Produkten;
- sehr geringer Halogengehalt.

Die Kombination von aliphatischen Urethan- und Acrylsegmenten in der Polymerstruktur schafft ein elastomeres Material, das die herausragenden chemischen Eigenschaften von Acrylaten mit der hohen mechanischen Festigkeit von aliphatischen Urethanharzen in sich vereinigt.

### Anwendungen:

Albiflex XP 6/1166 ist zur Verwendung in flexiblen Beschichtungen und Versiegelungs-Formulierungen in elektrischen/elektronischen Anwendungen besonders gut geeignet. Acrylische Klebstoffe können bezüglich Flexibilität optimiert werden, ohne dass Festigkeit und andere Eigenschaften verschlechtert werden.

### Formulierungen und Verarbeitung:

Albiflex XP 6/1166 kann mit fast allen radikalbildenden Photoinitiatoren und Peroxiden gehärtet werden. Albiflex XP 6/1166 kann auch mit lichtempfindlichen Prä-Addukten gehärtet werden. Albiflex XP 6/1166 zeigt gute Kompatibilität mit aliphatischen Urethane(meth)acrylaten, aromatischen Urethan(meth)acrylaten, Polyester(meth)acrylat-Harzen, Epoxy(meth)acrylaten, Amin(meth)acrylaten und Melamin (meth)acrylaten. Albiflex XP6/1166 kann zusammen mit Acrylatmonomeren, Acrylatoligomerenn, Lösemitteln usw. formuliert werden, um weitere Eigenschaftsmodifikationen der fertigen Produkte zu erreichen. Weitere Informationen zu Formullerungen kann auf Anfrage zur Verfügung gestellt werden. Formulierungszutaten, die nach Mischung mit Albiflex XP 6/1166 erhöhte Trübung zeigen, sollten gemieden werden, weil das ein Anzeichen von Inkompatibilität ist. Eventuelles Weißwerden während der Härtung ist jedoch kein problematisches Anzeichen, sondern weist auf eine Änderung des Brechungsindex hin.

### Lagerung:

Albiflex XP 6/1166 sollte an einem kühlen und trockenen Ort gelagert werden. Direktes Licht sollte während der Lagerung vermieden werden.

### Technische Daten:

### Erscheinungsbild:

Klare Flüssigkeit; Dichte bei 20 °C [g/ml]: ~ 1.0; Viskosität bei 25 °C [mPa·s]: 30,000 - 60,000; Haltbarkeit ungeöffnet 6 Monate.

Erfindungsgemäß soll es sich bei dem aliphatischen Urethanmethacrylat insbesondere nicht um das bekannte Dentalmonomer UDMA (ein Additionsprodukt aus 2-Hydroxyethylmethacrylat und 2,2,4-Hexamethylendiisocyanat) handeln. Dieses kann in den erfindungsgemäßen Zusammensetzungen in seiner üblichen Eigenschaft als weiteres Monomer, besonders als Vernetzer zugegen sein.

Der Anteil an aliphatischem Urethan(Meth)acrylat in den erfindungsgemäßen Zusammensetzungen beträgt beispielsweise 0,1 % bis 20 Gew.%, bevorzugt. 0,1 bis 10 Gew.%. Die %-Angaben verstehen sich bezogen auf die Gesamtzusammensetzung. Das aliphatische Urethan(Meth)acrylat kann in der flüssigen Monomerkomponente (A) oder in der Feststoffkomponente bzw. Pulverkomponente (B) oder in beiden zugegen sein. Bevorzugt befindet es sich in Komponente (A).

Zusätzlich können in den erfindungsgemäßen kalt- oder autopolymerisierenden Prothesenwerkstoffen ein oder mehrere Stoff(e) aus den Gruppen der weiteren Monomeren, Füllstoffe, Pigmente, Stabilisatoren, Regler, antimikrobiellen Additive, UV-Absorber, Thixotropiermittel, Katalysatoren und Vernetzer enthalten sein.

Als weitere Monomere kommen die auf dem Dentalgebiet üblichen Monomere in Betracht Beispiele sind radikalisch polymerisierbare monofunktionelle Monomere wie Mono(meth)-acrylate, Methyl-, Ethyl-, Butyl-, Benzyl-, Furfuryl- oder Phenyl(meth)acrylat, di- bzw. polyfunktionelle Monomere wie di- oder polyfunktionelle Acrylate bzw. Methacrylate, z.B. Bisphenol-A-di(meth)acrylat, Bis-GMA (ein Additionsprodukt aus Methacrylsäure und Bisphenol-A-diglycidylether), Di-, Tri- oder Tetraethylenglykoldi(meth)acrylat, Decandioldi(meth)acrylat, Dodecan-dioldi(meth)acrylat, Hexyldecandioldi(meth)acrylat, Trimethylolpropantri(meth)acrylat, Pentaerythrittetra(meth)-acrylat sowie Butandioldi(meth)acrylat, Ethylenglycol-di(meth)acrylat, Polyethylenglycol-di(meth)acrylate, ethoxylierte/propoxylierte Bisphenol-A-di(meth)acrylate.

Als Füllstoffe kommen z.B. pyrogene oder Fällungskieselsäuren, Dentalgläser wie Aluminosilicatgläser oder Fluoroaluminosilicatgläser, Strontiumsilicat, Strontiumborosilicat, Lithiumsilicat, Lithiumaluminiumsilicat, Schichtsilikate, Zeolithe, amorphe sphärische Füller auf Oxid- oder Mischoxidbasis (SiO₂, ZrO₂ und/oder TiO₂), Metalloxide mit Primärteilchengröße von ca. 40 bis 300 nm, Splitterpolymerisate mit 10- 100 µm Teilchengröße (vgl. R. Janda, Kunststoffverbundsysteme, VCH Verlagsgesellschaft, Weinheim, 1990, Seite 225 ff.) oder deren Mischungen in Frage. Zudem können Verstärkungsmittel wie Glasfasern, Polyamid- oder Kohlenstofffasern eingearbeitet werden.

Die Füllstoffe werden in der Regel zu 0 bis 10 Gew.%, vorzugsweise zu 0 bis 3 Gew. %, bezogen auf die gesamte Prothesenkunststoffzusammensetzung bzw. die Summe der Komponenten (A) und (B), eingesetzt.

Geeignete Stabilisatoren sind z. B. Hydrochinonmonomethylether oder 2,6-Di-tert.-butyl-4-methylphenol (BHT).

Des Weiteren können die erfindungsgemäßen Prothesenbasismaterialien übliche Zusatzstoffe enthalten, z.B. aus den Gruppen der antimikrobiellen Additive, UV-Absorber, Thixotropiermittel, Katalysatoren und Vernetzer.

Solche Additive werden - wie auch Pigmente, Stabilisatoren und Regler - in eher geringen Mengen eingesetzt, z. B. insgesamt zu 0,01 bis 3,0, besonders 0,01 bis 1,0 Gew.% bezogen auf die Gesamtmasse des Materials.

Die Pulverkomponente des Zweikomponenten-Prothesenbasismaterials enthält in der Regel ein Polymerpulver, insbesondere auf Methacrylatbasis, und/oder ein Perlpolymerisat auf Methacrylat-Basis. Perlpolymerisate werden auf diesem Gebiet häufig als Pulver bezeichnet.

Perlpolymerisate, insbesondere solche aus (Meth)acrylaten, sind dem Fachmann bekannt. Perlpolymerisate auf der Basis von Polyalkyl(meth)acrylaten erhält man in bekannter Weise durch Fällungspolymerisation oder Suspensionspolymerisation. Dabei liefert die Suspensionspolymerisation in der Regel größere Teilchen. Die durchschnittlichen Teilchengrößen decken eine weite Spanne ab und können beispielsweise von 0,1 µ bis 250 mm betragen. Es kann sich auch um vernetzte Perlpolymerisate handeln. Geeignete multifunktionelle Vernetzermoleküle sind der obigen Aufzählung der auf dem Dentalgebiet üblichen Monomeren zu entnehmen.

In das Perlpolymerisat können auch weitere Comonomere einpolymerisiert sein. Exemplarisch seien als monofunktionelle Comonomere genannt Styrol, alpha-Methylstyrol, Vinyltoluol, substituierte Vinyltoluole wie Vinylbenzylchloride, Butadien, Isobutylen, 2-Chlorbutadien, 2-Methylbutadien, Vinylpyridin, Cyclopenten, sowie (Meth)acryl-säureester wie Methylmethacrylat, Butylmethacrylat, Butylacrylat und Hydroxyethylmethacrylat, ferner Acrylnitril, Vinylacetat und Vinylpropionat sowie Mischungen dieser Comonomere. Zu den bevorzugten Comonomeren gehören unter anderen Vinylhalogenide wie Vinylchlorid, Vinylester, wie Vinylacetat, heterocyclische Vinylverbindungen wie 2-Vinylpyridin, Maleinsäure und Maleinsäurederivate, wie beispielsweise Maleinsäureanhydrid, Fumarsäure und Fumarsäurederivate wie Fumarsäureester, Acrylsäure, Methacrylsäure sowie Aryl(meth)acrylate wie Benzylmethacrytat oder Phenylmethacrylat.

In einer bevorzugten Ausgestaltung sind in die Perlen des ersten (Co)polymerisats und/oder in die Perlen des zweiten (Co)polymerisats zumindest teilweise Vernetzer einpolymerisiert worden. Die ersten und zweiten Perlpolymerisate umfassen somit auch vernetzte und teilvemetzte Perlpolymerisate.

Für die Vernetzung greift man regelmäßig auf multifunktionelle Comonomere oder auch multifunktionelle Oligomere zurück. Neben di-, tri- und polyfunktionellen (Meth)acrylaten eignen sich hierfür auch Pfropfvemetzer mit mindestens zwei unterschiedlichen reaktiven C-C-Doppelbindungen, beispielsweise Alkylmethacrylaten und Alkylacrylaten, sowie aromatische Vernetzer wie 1,2-Divinylbenzol, 1,3-Divinylbenzol und 1,4-Divinylbenzol. Unter den difunktionellen (Meth)acrylaten seien insbesondere die (Meth)acrylate des Propandiols, Butandiols, Hexandiols, Octandiols, Nonandiols, Decandiols und Eicosandiols sowie ferner die Di(meth)acrylate des Ethylenglycols, Triethylenglycols, Tetraethylenglycols, Dodecaethylenglycols, Tetradecaethylenglycols, Propylenglycols, Dipropylenglycols und Tetradecapropylenglycols, außerdem Glycerindi(meth)acrylat, 2,2-bis[(gamma-methacryloxy-beta-oxypropoxy)-phenylpropan], bis-GMA, Bisphenol-A-dimethacrylat, Neopentylglycol-di(meth)acrylat, 2,2-di-methacryloxypoly-ethoxyphenyl)propan mit 2 bis 10 Ethoxygruppen pro Molekül sowie 1,2-Bis(3-methacryloxy-2-hydroxypropoxy)butan genannt. Exemplarisch seien als multifunktionelle (Meth)acrylate, z. B. Di-, Tri- und/oder Tetra(meth)acrylate, wie 1,4-Butandioldimethacrylat, Ethylenglycoldimethacrylat sowie dl-oder trivinylische Verbindungen wie Divinylbenzol hervorgehoben.

Der Gehalt an derartigen Vernetzermolekülen liegt vorzugsweise im Bereich von 0,1 Gew.-% bis 10 Gew.-%, insbesondere im Bereich von 0,5 Gew.-% bis 5 Gew.-%, in der Ausgangsmischung für das Perlpoymerisat.

Als geeignete Alkylmethacrylate für die flüssige Komponente (A) sei auf Methyl-, Ethyl-, n-Propyl, i-Propyl-, n-Butyl, t-Butyl, i-Butyl, Benzyl- und Furfurylmethacrylate oder deren Mischungen verwiesen. Methylmethacrylat ist davon besonders bevorzugt.

In einer zweckmäßigen Ausgestaltung liegt in der flüssigen Komponente (A) neben mindestens einem der vorangehend genannten monofunktionellen Alkylmethacrylatmonomere mindestens ein Vernetzer vor. Dabei handelt es sich beispielsweise um multifunktionelle Monomere, Comonomere oder auch multifunktionelle Oligomere. Neben di-, tri- und polyfunktionellen (Meth)acrylaten eignen sich hierfür auch Pfropfvernetzer mit mindestens zwei unterschiedlichen reaktiven C-C-Doppelbindungen, beispielsweise Alkylmethacrylaten und Alkylacrylaten, sowie aromatische Vernetzer wie 1,2-Divinylbenzol, 1,3-Divinylbenzol und 1,4-Divinylbenzol. Unter den difunktionellen (Meth)acrylaten seien insbesondere die (Meth)acrylate des Propandiols, Butandiols, Hexandiols, Octandiols, Nonandiols, Decandiols und Eicosandiols sowie ferner die Di(meth)acrylate des Ethylenglycols, Triethylenglycols, Tetraethylenglycols, Dodecaethylenglycols, Tetradecaethylenglycols, Propylenglycols, Dipropylenglycols und Tetradecapropylenglycols, außerdem Glycerindi(meth)acrylat, 2,2-bis[(gamma-methacryloxy-beta-oxypropoxy)-phenylpropan], bis-GMA, Bisphenol-A-dimethacrylat, Neopentylglycol-di(meth)acrylat, 2,2-di-methacryloxypolyethoxyphenyl)propan mit 2 bis 10 Ethoxygruppen pro Molekül sowie 1,2-Bis(3-methacryloxy-2-hydroxypropoxy)butan genannt. Exemplarisch seien als multifunktionelle (Meth)acrylate, z. B. Di-, Tri- und/oder Tetra(meth)acrylate, wie 1,4-Butandioldimethacrylat, Ethylenglycoldimethacrylat sowie di-oder trivinylische Verbindungen wie Divinylbenzol hervorgehoben. Selbstverständlich können auch Mischungen der genannten Vernetzermoleküle zum Einsatz kommen. Besonders geeignet sind auch solche multifunktionellen Verbindungen, insbesondere di- und/oder tri-funktionelle Verbindungen, die elastische Einheiten besitzen und demgemäß geeignet sind, die aus den Prothesenausgangsmaterialien erhaltenen Prothesenmaterialien mit flexiblen Eigenschaften auszustatten.

Exemplarisch sei auf die Dimethacrylate wie 1,4-Butandiol-dimethacrylat verwiesen. Solche Vernetzermoleküle können in der Flüssigkomponente (A) in Mengen im Bereich von 0,1 bis 20 Gew.-%. vorzugsweise im Bereich von 1 bis 10 Gew.-%, beispielsweise 5 Gew.-% zugegen sein.

In einer weiteren Ausführungsform können in der Flüssigkomponente (A) neben z. B. Methylmethacrylat als bevorzugtem Hauptmonomer (> 50 Gew.-%) weitere Comonomere vorliegen.

Das für die Polymerisation erforderliche radikalische Initiatorsystem ist, je nach Reaktionsbedingungen bzw. Polymerisationssystem, in der flüssigen Komponente (A) und/oder der pulverförmigen Komponente (B) enthalten. Diesbezügliche Details sind dem Fachmann bekannt. Beispielsweise liegt bei Basismischungen für Kaltpolymerisate das Initiatorsystem meist in beiden Komponenten, der flüssigen Komponente und der pulverförmigen Komponente vor und wird demgemäß beim Mischen dieser Komponenten zusammengeführt. Folglich liegt in der Regel eine Initiatorkomponente (C) in der pulverförmigen Komponente (B) vor, insbesondere in Form von Peroxiden, Perketalen, Perestern und/oder Azoverbindungen. Ein weiterer Teil des Initiatorsystems (C) kann sich in der Flüssigkomponente (A) befinden, in der Regel ein Coinitiator. Als Initiatoren können auch Restgehalte an bei der Herstellung der pulverförmigen Komponenten nicht abreagierter Initiatorkomponente verwandt werden, z. B. Peroxide wie Dibenzoylperoxid.

Als Initiatoren für die Polymerisationsreaktion von kalt- bzw. autopolymerisierenden Ausgangsmischungen kommen grundsätzlich solche in Betracht, mit denen sich radikalische Polymerisationsreaktionen starten lassen. Bevorzugte Initiatoren sind Peroxide sowie Azoverbindungen, wie zum Beispiel die folgenden:
LPO: Dilauroylperoxid,
BPO: Dibenzoylperoxid,
t-BPEH: tert.-Butylper-2-ethylhexanoat,
AIBN: 2,2'-Azobis-(isobutyronitril),
DTBP: Di-tert.-butylperoxid.

Um die Initiierung der radikalischen Polymerisation durch Peroxide zu beschleunigen, können geeignete Aktivatoren, z. B. aromatische Amine, hinzugefügt werden. Exemplarisch seien als geeignete Amine N,N-Dimethyl-p-toluidin, N,N-Dihydroxyethyl-p-toluidln und p-Dibenzylaminobenzoesäurediethylester genannt. Hierbei fungieren die Amine regelmäßig als Co-Initiatoren und liegen üblicherweise in einer Menge von bis zu 0,5 Gew.-% vor.

Als radikalische Initiatorsysteme sind weiterhin Redoxsysteme geeignet, insbesondere Kombinationen aus Dibenzoylperoxid, Dilauroyl oder Campherchinon mit Aminen wie N,N-Dimethyl-p-toluidin, N-N-Dihydroxyethyl-p-toluidin und p-Dimethylaminobenzoesäurediethylester. Ferner können als Redoxsysteme auch solche zum Einsatz kommen, die neben einem Peroxid auch noch Ascorbinsäure oder deren Derivate, Barbitursäure oder ein Barbitursäurederivat oder eine Sulfinsäure als Reduktionsmittel enthalten. In einer bevorzugten Ausführungsform enthält ein solches Redoxsystem Barbitursäure oder Thiobarbitursäure oder ein Barbitursäure- oder Thiobarbitursäurederivat (beispielsweise 25 bis 80 Gew.-%), mindestens ein Kupfersalz oder ein Kupferkomplex (beispielsweise 0,1 bis 8 Gew.-%) und mindestens eine Verbindung mit einem ionogen vorliegenden Halogenatom (beispielsweise 0,05 bis 7 Gew.-%). Exemplarisch seien als geeignete Bestandteile des vorangehend genannten Redoxsystems 1-Benzyl-5-Phenylbarbitursäure, Kupferacetylacetonat und Benzyldibutylammoniumchlorid genannt.

Die Aushärtung der Zusammensetzungen erfolgt vorzugsweise durch redoxinduzierte radikalische Polymerisation bei Raumtemperatur bzw. bei leicht erhöhter Temperatur unter leichtem Druck, um Blasenbildung zu vermeiden. Als Initiatoren für die bei Raumtemperatur durchgeführte Polymerisation werden z.B. Redoxinitiator-Kombinationen, wie z.B. Kombinationen von Benzoyl- oder Laurylperoxid mit N,N-Dimethyl-sym.-xylidin oder N,N-Dimethyl-p-toluidin, verwendet.

Bevorzugt enthält die Komponente (C) Barbitursäure oder ein Barbitursäurederivat.

Ein besonders bevorzugtes Initiatorsystem ist eine Kombination von Barbitursäuren in Verbindung mit Kupfer- und Chlorid-lonen sowie oben genannten Peroxiden. Dieses System zeichnet sich durch eine hohe Farbstabilität aus.

Ferner kann man die pulverförmige Komponente (B) und/oder die Flüssigkomponente (A) in bekannter Weise mit weiteren Zusatzstoffen aus den Gruppen der Stabilisatoren, UV-Absorbern, Thixotropiermitteln und Füllstoffen versehen.

Des Weiteren können die pulverförmige Komponente (B) und/oder die Flüssigkomponente (A) mit weiteren Additiven, beispielsweise antimikrobiellen Additiven, ausgestattet sein. Solche geeigneten biozidwirkenden Additive können z. B., insbesondere in Form einer Mischung, mindestens ein anorganisches Kupfersalz, mindestens ein anorganisches Silbersalz und mindestens ein anorganisches Bariumsalz sowie optional Silber umfassen. Dabei ist bevorzugt, dass das Kupfersalz Kupfersulfat, das Silbersalz Silberphosphat und das Barlumsalz Bariumsulfat darstellen. Gemäß einer weiteren, bevorzugten Ausgestaltung ist vorgesehen, dass das anorganische Kupfersalz, insbesondere Kupfersulfat, und/oder das anorganische Silbersalz, insbesondere Silberphosphat, und/oder das anorganische Bariumsalz, insbesondere Bariumsulfat, und/oder gegebenenfalls Silber, auf einem inerten Trägermaterial vorliegt bzw. vorliegen. Vorzugsweise liegen das anorganische Kupfersalz, insbesondere Kupfersulfat, das anorganische Silbersalz, insbesondere Silberphosphat, und das anorganische Bariumsalz, insbesondere Bariumsulfat, sowie gegebenenfalls Silber gemeinsam auf einem Träger vor. Geeignete inerte Trägermaterialen umfassen z. B. in partikulärer Form vorliegende Polymermaterialien und auch anorganische Substanzen, z. B. Silikate.

Der erfindungsgemäße Werkstoff eignet sich besonders als Prothesenbasismaterial, kommt aber auch im Veterinärbereich für Hufreparaturmaterialien, für Knochenzement zur Zementierung von künstlichen Gelenkprothesen und für kieferorthopädischen Apparaturen in Frage.

Die folgenden Beispiele erläutern die Erfindung näher, ohne dass sie darauf beschränkt werden soll. Teile- und Prozentangaben beziehen sich wie in der übrigen Beschreibung auf das Gewicht, sofern nicht anders angegeben.

### Ausführungsbeispiel 1a: Herstellen einer Pulvermischung (A):

Aus PMMA-Perlen unterschiedlicher Korngröße wird unter Zusatz von Barbitursäure eine Mischung hergestellt.

### Ausführungsbeispiel 1b: Herstellen einer erfindungsgemäßen Flüssigkeit/Monomermischung (B):

Aus Methylmethacrylat, Butandioldimethacrylat und einem weiteren methacrylatbasierten Vernetzer wird unter Zusatz von Stabilisatoren und Initiatoren (Barbitursäure / Kupfer - System) eine Mischung hergestellt. Ferner wird noch das erfindungsgemäße aliphatische Urethandiacrylat zugesetzt, in diesem Fall Albiflex® XP6/1166 der Fa. Nano Resins /Geesthacht, Deutschland.

### Ausführungsbeispiel 2: Herstellen von Prüfkörpern, Kochtest, Bestimmung der Farbwerte, Bestimmung der mechanischen Eigenschaften:

Pulvermischung und Monomermischung werden im Verhältnis 10:7 intensiv gemischt und nach der Anquellphase Prüfkörper mit einer Abmessung von 30 x 30 x 3 mm gegossen.

Als Referenz 2 werden Prüfkörper aus einer Monomermischung, welche kein aliphatisches Urethandiacrylat enthält, und dem gleichen Pulver, gegossen. Ferner werden als Referenz 1 Prüfkörper mit einem Butadien-Acrylonitril-Oligomer als Additiv hergestellt (vgl. EP1923037A2).

Die Prüfkörper werden zunächst farbmetrisch (Gerät Spektralphotometer Datacolor® SF 600) vermessen und anschließend für 8 Wochen in VE-Wasser bei 50 °C gelagert. Anschließend wird wieder die Farbe gemessen und die Veränderung des b-Wertes und E-Wertes bestimmt. Mit der erfindungsgemäßen Monomermischung und den Referenzmischungen wird ebenfalls Material zur Bestimmung von Biegefestigkeit, E-Modul und Bruchzähigkeit hergestellt. Die Ergebnisse sind der folgenden Tabelle zu entnehmen.

| | Biegefestigkeit [MPa] | E-Modul [MPa] | Brucharbeit [J/m²] | Bruchintensitätsfaktor [MPa*m^{1/2}] | Delta b (ΔGelbwert) nach 8 Wochen Lagerung bei 50 °C in Wasser | Delta E (ΔGesamtver färbung) nach 8 Wochen Lagerung bei 60 °C in Wasser |
|---|---|---|---|---|---|---|
| Erfindungsgemäße Monomermischung | 70 | 2097 | 457 | 1,99 | 0,28 | 0,44 |
| Referenz 1 (mit Butadien-Acrylonitril-Oligomer Additiv) | 72 | 2160 | 642 | 2,33 | 5,42 | 5,68 |
| Referenz 2 (ohne Additiv) | 75 | 2346 | 274 | 1,71 | 0,35 | 0,36 |

### Erläuterung der Werte:

Die Referenz 2 ohne Additiv zeigt eine geringe Verfärbungsneigung, allerdings auch keine verbesserte Bruchresistenz.

Mischungen enthaltend ein elastifizierendes Additiv für hohe Bruchresistenz (Referenz 1) neigen allerdings zur Vergilbung und zur Verfärbung.

Die erfindungsgemäße Monomermischung zeigt überraschenderweise sowohl eine verbesserte Bruchzähigkeit als auch eine geringe Verfärbungsneigung. Damit ist es sie Prothesenbasismaterial sehr gut geeignet.

## Patentansprüche

1. Prothesenbasismaterial, erhalten aus einer autopolymerisierenden oder kaltpolymerisierenden Zusammensetzung, die
(A) eine flüssige Monomerkomponente,
(B) eine pulverförmige Komponente, die ein oder mehrere Perlpolymerisat(e) umfasst,
(C) mindestens einen Initiator oder ein Initiatorsystem für die Auto- oder Kaltpolymerisation
enthält, **dadurch gekennzeichnet, dass** in den Komponenten (A) oder/und (B) mindestens ein Mitglied der Gruppen der aliphatischen Urethanacrylate und der aliphatischen Urethanmethacrylate vorhanden ist, wobei die Komponente (A) mindestens ein flüssiges aliphatisches Urethandiacrylat-Harz umfasst und die aliphatischen Urethan(meth)acrylate Polymere mit aliphatischen Urethansegmenten und (Meth)Acrylatsegmenten sind.

2. Prothesenbasismaterial nach Anspruch 1, **dadurch gekennzeichnet, dass** die Zusammensetzung frei von elastifizierendem synthetischen Kautschuk ist.

3. Prothesenbasismaterial nach Anspruch 1, **dadurch gekennzeichnet dass** es nach Härtung eine Bruchzähigkeit von > 1,8 MPa*m^{1/2} und eine Brucharbeit von > 400 J/m² aufweist.

4. Prothesenbasismaterial gemäß Anspruch 1 oder Anspruch 2, **dadurch gekennzeichnet, dass** es nach 8 Wochen Lagern in vollentsalztem Wasser bei 50°C eine Differenz im Gelbwert, dem b-Wert, von maximal einer Einheit aufweist.

5. Prothesenbasismaterial nach einem der vorstehenden Ansprüche, **dadurch gekennzeichnet, dass** der Anteil an aliphatischem Urethanacrylat und/oder Urethanmethacrylat 0,5 bis 20 Gew. % beträgt.

6. Prothesenbasismaterial nach Anspruch 5, **dadurch gekennzeichnet, dass** der Anteil an aliphatischem Urethanacrylat und/oder Urethanmethacrylat 0,1 bis 5 Gew. % beträgt.

7. Prothesenbasismaterial nach einem der vorstehenden Ansprüche, **dadurch gekennzeichnet, dass** das aliphatische Urethanacrylat und/oder Urethanmethacrylat in der Polymerstruktur aliphatische Urethansegmente und (Meth)Acrylatsegmente aufweist.

8. Prothesenbasismaterial nach einem der vorstehenden Ansprüche , **dadurch gekennzeichnet, dass** das aliphatische Urethanacrylat und/oder Urethanmethacrylat farblos und transparent ist.

9. Prothesenbasismaterial nach einem der vorstehenden Ansprüche, **dadurch gekennzeichnet, dass** das aliphatische Urethanacrylat und/oder Urethanmethacrylat keinen Eigengeruch aufweist.

10. Prothesenbasismaterial nach einem der vorstehenden Ansprüche, erhalten aus einer autopolymerisierenden oder kaltpolymerisierenden Zusammensetzung enthaltend die Komponenten (A), (B) und (C), die zusätzlich einen oder mehrere Stoffe aus den Gruppen der Füllstoffe, Pigmente, Stabilisatoren, Regler, antimikrobiellen Additive, UV-Absorber, Thixotropiermittel, Katalysatoren, weiteren Monomere und Vernetzer enthält.

11. Prothesenbasismaterial nach einem der vorstehenden Ansprüche zur Verwendung für Hufreparaturmaterialien im Veterinärbereich, für Knochenzement zur Zementierung von künstlichen Gelenkprothesen, oder für kieferorthopädische Apparaturen.

12. Verwendung eines Mitglieds der Gruppen der aliphatischen Urethanacrylate und der aliphatischen Urethanmethacrylate als Modifikator in Zusammensetzungen für auto- oder kaltpolymerisierendes Prothesenbasismaterial, wobei es sich bei dem Modifikator um ein flüssiges aliphatisches Urethandiacrylat-Harz, das sich aus aliphatischen Urethan- und Acrylatsegmenten zusammensetzt, handelt und die aliphatischen Urethan(meth)acrylate Polymere mit aliphatischen Urethan- und (Meth)Acrylatsegmenten sind.

13. Verwendung nach Anspruch 12, wobei die Zusammensetzungen frei von elastifizierendem synthetischen Kautschuk sind.

14. Verwendung nach einem der vorstehenden Ansprüche 12 oder 13, **dadurch gekennzeichnet, dass** keine negative Beeinflussung der Resultate des Suntests nach ISO 20795 erfolgt.

## Claims

1. Denture base material, obtained from an
auto-polymerising or cold-polymerising composition containing
(A) a liquid monomer component,
(B) a powdered component comprising one or more bead polymer(s),
(C) at least one initiator or one initiator system for auto- or cold-polymerisation, **characterised in that** at least one member of the groups of aliphatic urethane acrylates and aliphatic urethane methacrylates is present in components (A) or/and (B), component (A) comprising at least one liquid aliphatic urethane diacrylate resin and the aliphatic urethane (meth)acrylates being polymers with aliphatic urethane segments and (meth)acrylate segments.

2. Denture base material according to claim 1, **characterised in that** the composition is free from plasticising synthetic rubber.

3. Denture base material according to claim 1, **characterised in** having a fracture toughness of > 1.8 MPa*m^{1/2} and a fracture work of > 400 J/m² after curing.

4. Denture base material according to claim 1 or claim 2, **characterised in** having a difference in the yellow value, the b value, of a maximum of one unit after 8 weeks of storage in totally demineralised water at 50°C.

5. Denture base material according to any one of the preceding claims, **characterised in that** the content of aliphatic urethane acrylate and/or urethane methacrylate is 0.5 to 20 % by weight.

6. Denture base material according to claim 5, **characterised in that** the content of aliphatic urethane acrylate and/or urethane methacrylate is 0.1 to 5 % by weight.

7. Denture base material according to any one of the preceding claims, **characterised in that** the aliphatic urethane acrylate and/or urethane methacrylate has aliphatic urethane segments and (meth)acrylate segments in the polymer structure.

8. Denture base material according to any one of the preceding claims, **characterised in that** the aliphatic urethane acrylate and/or urethane methacrylate is colourless and transparent.

9. Denture base material according to any one of the preceding claims, **characterised in that** the aliphatic urethane acrylate and/or urethane methacrylate has no inherent odour.

10. Denture base material according to any one of the preceding claims, obtained from an auto-polymerising or cold-polymerising composition containing components (A), (B), and (C) which additionally contains one or more substances from the groups of fillers, pigments, stabilisers, modifiers, antimicrobial additives, UV-absorbing agents, thixotroping agents, catalysts, further monomers, and cross-linkers.

11. Denture base material according to any one of the preceding claims for use for hoof repair materials in the veterinary field, for bone cement for cementing artificial joint prostheses, or for orthodontic appliances.

12. Use of a member of the groups of aliphatic urethane acrylates and aliphatic methacrylates as modifying agent in compositions for auto- or cold-polymerising denture base material, the modifying agent being a liquid aliphatic urethane diacrylate resin composed of aliphatic urethane segment and acrylate segments, and the aliphatic urethane (meth)acrylates being polymers with aliphatic urethane segments and (meth)acrylate segments.

13. Use according to claim 12, wherein the compositions are free from plasticising synthetic rubber.

14. Use according to any one of preceding claims 12 or 13, **characterised in that** there is no negative influence on the results of the Suntest according to ISO 20795.

## Revendications

1. Matériau de base prothétique, obtenu d'un composition autopolymérisant ou polymérisant à froid contenant
(A) au moins un composant monomère liquide,
(B) au moins un composant pulvérulent comprenant un ou plusieurs polymère(s) en perles,
(C) au moins un initiateur ou un système d'initiateurs pour l'autopolymérisation ou la polymérisation à froid,
**caractérisé en ce qu'**au moins un membre des groupes des acrylates d'uréthane aliphatiques et des méthacrylates d'uréthane aliphatiques est présent dans les composants (A) ou/et (B), le composant (A) comprenant au moins une résine de diacrylate d'uréthane aliphatique liquide et les (méth)acrylates d'uréthane aliphatiques étant des polymères avec des segments d'uréthane aliphatiques et des segments de (méth)acrylate aliphatiques.

2. Matériau de base prothétique selon la revendication 1, **caractérisé en ce que** la composition est exempte du caoutchouc synthétique plastifiant.

3. Matériau de base prothétique selon la revendication 1, caractérisé en ayant une ténacité à la rupture de > 1.8 MPa*m^{1/2} and une énergie à la rupture de > 400 J/m² après durcissement.

4. Matériau de base prothétique selon la revendication 1 ou 2, caractérisé en ayant une différence de la valeur jaune, le valeur b, d'un maximum d'une unité après 8 semaines du stockage dans l'eau totalement déminéralisée à 50°C.

5. Matériau de base prothétique selon l'une des revendications précédentes, **caractérisé en ce que** la teneur de l'acrylate d'uréthane aliphatique et/ou du méthacrylate d'uréthane aliphatique est 0,5 à 20 % en poids.

6. Matériau de base prothétique selon la revendication 5, **caractérisé en ce que** la teneur de l'acrylate d'uréthane aliphatique et/ou du méthacrylate d'uréthane aliphatique est 0,1 à 5 % en poids.

7. Matériau de base prothétique selon l'une des revendications précédentes, **caractérisé en ce que** l'acrylate d'uréthane aliphatique et/ou le méthacrylate d'uréthane aliphatique a des segments d'uréthane aliphatiques et des segments de (méth)acrylate aliphatiques dans la structure polymère.

8. Matériau de base prothétique selon l'une des revendications précédentes, **caractérisé en ce que** l'acrylate d'uréthane aliphatique et/ou le méthacrylate d'uréthane aliphatique est incolore et transparent.

9. Matériau de base prothétique selon l'une des revendications précédentes, **caractérisé en ce que** l'acrylate d'uréthane aliphatique et/ou le méthacrylate d'uréthane aliphatique n'a pas d'odeur inhérente.

10. Matériau de base prothétique selon l'une des revendications précédentes, obtenu d'une composition autopolymérisant ou polymérisant à froid contenant les composants (A), (B), et (C) qui contient en outre une ou plusieurs substances des groupes des charges, des pigments, des stabilisateurs, des modificateurs, des additifs antimicrobiens, des agents absorbant les UV, des agents thixotropes, des catalyseurs, des autres monomères, et des agents de réticulation.

11. Matériau de base prothétique selon l'une des revendications précédentes pour l'utilisation pour des matériaux de réparation de sabots dans le domaine vétérinaire, pour le ciment osseux pour cimenter des prothèses articulaires artificielles, ou pour des appareils orthodontiques.

12. Utilisation d'un membre des groups des acrylates d'uréthane aliphatiques et des méthacrylates d'uréthane aliphatiques comme agent modificateur dans des compositions pour un matériau de base prothétique autopolymérisant ou polymérisant à froid, l'agent modificateur étant une résiné de diacrylate d'uréthane aliphatique liquide composée des segments d'uréthane aliphatiques et des segments d'acrylate aliphatiques, et les (méth)acrylates d'uréthane aliphatiques étant des polymères avec des segments d'uréthane aliphatiques et des segments de (méth)acrylate aliphatiques.

13. Utilisation selon la revendication 12, selon laquelle la composition est exempte du caoutchouc synthétique plastifiant.

14. Utilisation selon l'une des revendications 12 ou 13 précédentes, **caractérisée en ce qu'**il n'y a pas d'influence négative sur les résultats du Suntest selon ISO 20795.
